# EUROPÄISCHE PATENTANMELDUNG

(11) **EP 0 586 909 A2**
(43) Veröffentlichungstag der Anmeldung: **16.03.1994**
(21) Anmeldenummer: 93112844.1
(22) Anmeldetag: 11.08.1993
(51) Int. Cl.: A61K 37/64

(54) **Verwendung von C1-Inaktivator zur Herstellung eines Arzneimittels zur Prophylaxe und Therapie des Capillary Leak Syndroms und Kreislaufschocks**

(30) Priorität: 24.08.1992 DE 4227762
(71) Anmelder: BEHRINGWERKE Aktiengesellschaft, 35001 Marburg (DE)
(72) Erfinder: Eisele, Bernd, D-35095 Lahntal (DE); Jessel, Andreas, D-35039 Marburg (DE); Delvos, Ulrich, D-35396 Giessen-Wieseck (DE)

(57) **Zusammenfassung**

Es wird die Verwendung von C1-Inaktivator zur Herstellung eines Arzneimittels zur Prophylaxe und Therapie des Capillary Leak Syndroms (generalisierte Extravasation) und Kreislaufschocks (refraktäre Hypotension) bei schweren Verbrennungen oder Verbrühungen, bei Polytraumata, Operationen unter Bedingungen des extrakorporalen Kreislaufs oder bei Anwendung von Zytokinen, endogenen Mediatoren, gentechnologisch produzierten Mediator-Hybriden oder Wachstumsfaktoren im Rahmen der therapeutischen Anwendung dieser Substanzen sowie des Capillary Leak Syndroms und des Veno-occlusive Disease der Leber nach therapeutisch oder prophylaktisch indizierter Knochenmarktransplantation beschrieben.

## Beschreibung

Die Erfindung betrifft die Verwendung von C1-Inaktivator zur Herstellung eines Arzneimittels zur Prophylaxe und Therapie des Capillary Leak Syndroms (generalisierte Extravasation) und Kreislaufschocks (refraktäre Hypotension) bei schweren Verbrennungen oder Verbrühungen, bei Polytraumata, Operationen unter Bedingungen des extrakorporalen Kreislaufs oder bei Anwendung von Zytokinen, endogenen Mediatoren, gentechnologisch produzierten Mediator-Hybriden oder Wachstumsfaktoren im Rahmen der therapeutischen Anwendung dieser Substanzen sowie des Capillary Leak Syndroms und des Veno-occlusive Disease der Leber (Lebervenenverschlußkrankheit) nach therapeutisch oder prophylaktisch indizierter Knochenmarktransplantation.

Das Kallikrein-System des Menschen besteht aus einer Reihe von Proteasen und Intermediärprodukten, die nach initialer Aktivierung sukzessive zur Bildung vasoaktiver Kinine (z. B. Bradykinin) führen. Die entscheidende Größe stellt die Menge an proteolytisch aktivem Kallikrein dar.

Die Aktivierung des Kallikrein-Systems kann durch direkte Einwirkung der schädigenden Mechanismen geschehen, aber auch mittelbar durch die Generierung von C-reaktivem Protein im Rahmen einer Akut-Phase-Reaktion, evtl. unter Zwischenschaltung weiterer Proteasen-Systeme.

Doch unabhängig von den zugrundeliegenden Triggermechanismen ist der weitere Ablauf des pathophysiologischen Geschehens abhängig von der Fähigkeit des Organismus, die Bildung von Kallikrein zu regulieren und die proteolytische Aktivität des generierten Kallikreins durch suffiziente Degradation bzw. Inhibition in ausreichendem Maße zu kontrollieren.

Der unter in-vivo-Bedingungen wichtigste physiologische Regulator des Kallikrein-Systems ist C1-Inaktivator. Er entfaltet seine Wirkung an der zentralen Stelle des Systems durch Interaktion mit der aktivierten Protease Kallikrein und deren Inhibition.

Die Aktivierung des Kallikrein-Systems wird im Verlauf einer Reihe von Erkrankungen sowie im Verlauf von therapeutischen und/oder prophylaktischen iatrogenen Interventionen beobachtet. Diese Aktivierungsprozesse sind allgemein mit einem Verbrauch der beteiligten Faktoren, insbesondere des Inhibitors, verbunden.

Obwohl durch die zu beobachtende Akut-Phase-Reaktion eine Mehrproduktion an C1-Inaktivator zu beobachten ist, reicht dessen inhibitorische Kapazität nicht aus, die Aktivierung des Kallikrein-Systems zu kontrollieren.

Die Ätiopathogenese des Schockgeschehens und des generalisierten Ödems bei Patienten mit schweren Verbrennungen stellt sich wie folgt dar: das thermische Trauma führt zu einer Akut-Phase-Reaktion und zur (mittelbaren und/oder unmittelbaren) Aktivierung des Kallikrein-Systems. Wird das Inhibitorpotential des Organismus verbraucht, kommt es zur ungehinderten Aktivierung und unkontrollierten Freisetzung von toxischen Intermediärprodukten (z. B. Kallikrein) sowie von ebenfalls potentiell schädigenden Endprodukten (Kinine, vor allem (v.a.) Bradykinin). Dies dokumentiert sich u. a. in der Erkenntnis, daß bei Patienten mit thermischem Trauma das Substrat "Präkallikrein" (als Maß für die Bildung von Kallikrein und Bradykinin) in Abhängigkeit von der Schwere des thermischen Traumas vermindert ist. Die genannten Substanzen haben das Potential, das Gefäßendothel direkt zu beeinflussen. Sie können aber auch durch Aktivierung von Sekundärmechanismen (z. B. NO [Stickoxyd], cGMP [zyklisches Guanosin Monophosphat]) entweder an der Endothelzellwand oder im Endothel selbst zu mittelbaren Schädigungen führen. Die pathomorphologische Folge der schädigenden Einwirkungen am Endothel sind eine Extravasation aus dem Gefäßsystem und/oder das verminderte Ansprechen der Gefäßmuskulatur auf gefäßverengende Impulse. Klinische Symptome beim Patienten sind das generalisierte Ödem und die refraktäre Hypotension (Kreislaufschock).

Pathogenetisch haben die genannten Substanzen das Potential, das Gefäßendothel direkt zu beeinflussen. Sie können aber auch durch Aktivierung von Sekundärmechanismen (z. B. NO, cGMP) entweder an der Endothelzellwand oder im Endothel selbst zu mittelbaren Schädigungen führen. Die pathomorphologische Folge der schädigenden Einwirkungen am Endothel sind eine Extravasation aus dem Gefäßsystem und/oder das verminderte Ansprechen der Gefäßmuskulatur auf gefäßverengende Impulse. Klinische Symptome beim Patienten sind das generalisierte Ödem und die refraktäre Hypotension (Kreislaufschock).

Die Ätiopathogenese des Schockgeschehens bei Patienten mit Polytrauma stellt sich wie folgt dar: das Trauma selbst bzw. die Folgeschäden im Organismus (instabile Frakturen, nekrotisches Gewebe) führen zu einer Akut-Phase-Reaktion und zur (mittelbaren und/oder unmittelbaren) Aktivierung des Kallikrein-Systems. Wird das Inhibitorpotential des Organismus verbraucht, kommt es zur ungehinderten Aktivierung und unkontrollierten Freisetzung von toxischen Intermediärprodukten (z. B. Kallikrein) sowie von ebenfalls potentiell schädigenden Endprodukten (Kinine, v.a. Bradykinin). Diese Substanzen haben das Potential, das Gefäßendothel direkt zu beeinflussen. Sie können aber auch durch Aktivierung von Sekundärmechanismen (z. B. NO, cGMP) entweder an der Endothelzellwand oder im Endothel selbst zu mittelbaren Schädigungen führen. In diagnostischen Untersuchungen findet sich bei Patienten mit Polytrauma das Substrat "Präkallikrein" (als Maß für die Bildung von Kallikrein und Bradykinin) vermindert. Das Ausmaß der Verminderung korreliert mit der Schwere der Verletzung. Die pathomorphologische Folge der schädigenden Einwirkungen am Endothel sind eine Extravasation aus dem Gefäßsystem, vor allem aber das verminderte Ansprechen der Gefäßmuskulatur auf gefäßverengende Impulse. Das vorherrschende klinische Symptom beim Patienten ist die refraktäre Hypotension (Kreislaufschock).

Die Ätiopathogenese des Schockgeschehens und des generalisierten Ödems bei Patienten im Zustand nach therapeutisch oder prophylaktisch indizierter Knochenmarktransplantation stellt sich wie folgt dar: die ablative Vorbehandlung (Chemotherapie und/oder Radiotherapie) führt allein oder auch im Zusammenspiel mit der darauffolgenden Knochenmarktransplantation zu einer Akut-Phase-Reaktion und zur (mittelbaren und/oder unmittelbaren) Aktivierung des Kallikrein- Systems. Wird das Inhibitorpotential des Organismus verbraucht, kommt es zur ungehinderten Aktivierung des Systems mit unregulierter Freisetzung von toxischen Intermediärprodukten (z. B. Kallikrein) sowie von ebenfalls potentiell schädigenden Endprodukten (Kinine, v.a. Bradykinin). Diese Substanzen haben das Potential, das Gefäßendothel direkt zu beeinflussen. Sie können aber auch durch Aktivierung von Sekundärmechanismen (z. B. NO, cGMP) entweder an der Endothelzellwand oder im Endothel selbst zu mittelbaren Schädigungen führen. Die pathomorphologische Folge der schädigenden Einwirkungen am Endothel sind eine Extravasation aus dem Gefäßsystem und/oder das verminderte Ansprechen der Gefäßmuskulatur auf gefäßverengende Impulse. Klinische Symptome beim Patienten sind das sog. "Veno-occlusive disease", das generalisierte Ödem und die refraktäre Hypotension (Kreislaufschock) und/oder eine progrediente Dysfunktion eines oder mehrerer Organsysteme.

Aus Diagnostikstudien am Menschen ist bekannt, daß das Auftreten von lebensbedrohlichen Komplikationen (generalisiertes Ödem und/oder Kreislaufschock) verbunden ist mit den Zeichen der dysregulierten Aktivierung des Kallikrein-Systems. Komplikationsfreie Intervalle gehen mit einer Normalisierung der Laborparameter einher.

Die Ätiopathogenese des Schockgeschehens und des generalisierten Ödems bei Patienten, die sich Operationen unter den Bedingungen eines extrakorporalen Kreislaufs (Bubble- oder Membran-Oxygenatoren) unterziehen, stellt sich wie folgt dar: der Kontakt des Blutes mit den Fremdoberflächen resp. dessen Interaktion mit den Sauerstoffblasen (bubbles) des Oxygenators führen zu einer Akut-Phase-Reaktion und zur (mittelbaren und/oder unmittelbaren) Aktivierung des Kallikrein-Systems. Wird das Inhibitorpotential des Organismus verbraucht, kommt es zur ungehinderten Aktivierung und unkontrollierten Freisetzung von toxischen Intermediärprodukten (z. B. Kallikrein) sowie von ebenfalls potentiell schädigenden Endprodukten (Kinine, v.a. Bradykinin). Die genannten Substanzen haben das Potential, das Gefäßendothel direkt zu beeinflussen. Sie können aber auch durch Aktivierung von Sekundärmechanismen (z. B. NO, cGMP) entweder an der Endothelzellwand oder im Endothel selbst zu mittelbaren Schädigungen führen. Die pathomorphologische Folge der schädigenden Einwirkungen am Endothel sind eine Extravasation aus dem Gefäßsystem und/oder das verminderte Ansprechen der Gefäßmuskulatur auf gefäßverengende Impulse. Klinische peri- und postoperative Symptome beim Patienten sind das generalisierte Ödem und/oder die refraktäre Hypotension (Kreislaufschock) und/oder eine Reduktion der kardialen Auswurfleistung (Cardiac output).

Die Ätiopathogenese des Schockgeschehens und des generalisierten Ödems bei Patienten unter Applikation von Zytokinen, endogenen Mediatoren, gentechnologisch produzierten Mediator-Hybriden und Wachstumsfaktoren im Rahmen der therapeutischen Anwendung dieser Substanzen einzeln, in Kombination untereinander oder in Kombination mit anderen therapeutischen oder prophylaktischen Maßnahmen stellt sich wie folgt dar: die Applikation dieser o.g. Stoffe, einzeln, in Kombination untereinander oder in Kombination mit anderen therapeutischen oder prophylaktischen Maßnahmen führt zu einer Akut-Phase-Reaktion und zur (mittelbaren und/oder unmittelbaren) Aktivierung des Kallikrein-Systems. Wird das Inhibitorpotenital des Organismus verbraucht, kommt es zur ungehinderten Aktivierung und unkontrollierten Freisetzung von toxischen Intermediärprodukten (z. B. Kallikrein) sowie von ebenfalls potentiell schädigenden Endprodukten (Kinine, v.a. Bradykinin).

Das pathogenetische Potential dieser Substanzen besteht darin, das Gefäßendothel direkt zu beeinflussen. Sie können aber auch durch Aktivierung von Sekundärmechanismen (z. B. NO, cGMP) entweder an der Endothelzellwand oder im Endothel selbst zu mittelbaren Schädigungen führen. Daneben aber können einige der o. g. Zytokine, endogenen Mediatoren und Wachstumsfaktoren (z. B. Interleukin-1ß, Tumor Nekrose Faktor, und Interferone) auch direkt auf das Endothel schädigend einwirken. Andere aber auch mittelbar dadurch, daß sie eine vermehrte Produktion bzw. Aktivierung der direkt schädigenden Zytokine induzieren. Bei einigen der Zytokine, endogenen Mediatoren und Wachstumsfaktoren konnte der genaue Pathomechanismus der Endothelschädigung noch nicht geklärt werden. Die pathomorphologische Folge der schädigenden Einwirkungen am Endothel sind eine Extravasation aus dem Gefäßsystem und/oder das verminderte Ansprechen der Gefäßmuskulatur auf gefäßverengende Impulse. Klinische Symptome beim Patienten sind das generalisierte Ödem und die refraktäre Hypotension (Kreislaufschock).

Demnach haben schwere Verbrennungen, Polytraumata, therapeutisch oder prophylaktisch indizierte Knochenmarktransplantationen, Operationen unter Bedingungen des extrakorporalen Kreislaufs sowie die therapeutische oder prophylaktische Anwendung von Zytokinen, endogenen Mediatoren, gentechnologisch produzierten Mediator-Hybriden und Wachstumsfaktoren als begleitende Erscheinung das generalisierte Ödem und die refraktäre Hypotension (Kreislaufschock) gemeinsam. Die genannten Symptome werden häufig nach Aktivierung des Kallikrein-Systems gesehen.

Wir fanden nun, daß die Applikation von C1-Inaktivator eine positive Wirkung bei der Therapie des generalisierten Ödems, der refraktären Hypotension (Kreislaufschock), der progredienten Dysfunktion von Organsystemen und der reduzierten Herzauswurfleistung (bei Patienten unter extrakorporaler Zirkulation) hat.

Gegenstand der Erfindung ist demnach die Verwendung von C1-Inaktivator zur Herstellung eines Arzneimittels zur Prophylaxe und Therapie des Capillary Leak Syndroms (generalisierte Extravasation) und Kreislaufschocks (refraktäre Hypotension) bei schweren Verbrennungen oder Verbrühungen, bei Polytraumata, Operationen unter Bedingungen des extrakorporalen Kreislaufs oder bei Anwendung von Zytokinen, endogenen Mediatoren, gentechnologisch produzierten Mediator-Hybriden oder Wachstumsfaktoren im Rahmen der therapeutischen Anwendung dieser Substanzen sowie des Capillary Leak Syndroms und des Veno-occlusive Disease der Leber nach therapeutisch oder prophylaktisch indizierter Knochenmarktransplantation.

Es kann C1-Inaktivator verwendet werden, der auf eine dem Fachmann bekannte Weise aus Blutplasma präpariert werden kann, und vorzugsweise als gereinigtes Produkt.

Der C1-Inaktivator ist als Arzneimittel als pyrogenfreies, gefriergetrocknetes Lyophilisat bekannt, das vor der Applikation gelöst und vorzugsweise intravenös injiziert wird.
Eine Einheit des C1-Inaktivator-Konzentrats entspricht der Aktivität von 1 ml gepooltem menschlichem Zitratplasma (1 Einheit [1 E] entspricht damit 6 Levy & Lepow-Einheiten).

Auch auf gentechnischem Wege exprimierter und gereinigter C1-Inaktivator kann zur Herstellung des Arzneimittels eingesetzt werden.

Das Arzneimittel kann für eine intravenöse (Bolus oder Infusion), intramuskuläre oder subkutane Applikation hergestellt werden.

Das Arzneimittel enthält 1 - 5.000 E/kg Körpergewicht (KG)/Tag, bevorzugt 5 - 1.000 E/kg KG/Tag C1-Inaktivator.

Für Erwachsene kann auch ein festes Arzneimittel mit einer Dosierung von 1 - 300.000 E/Tag, bevorzugt: 50 - 60.000 E/Tag C1-Inaktivator hergestellt werden.

C1-Inaktivator kann separat oder als Kombination mit anderen Arzneistoffen verwendet werden. Besonders in Kombination mit galenischen Hilfsstoffen ist auch die Herstellung einer peroralen oder rektalen Form möglich.

### Beispiel für klinische Anwendungen des erfindungsgemäßen Arzneimittels:

Bei Patienten mit generalisiertem Ödem und refraktärer Hypotension (Kreislaufschock) im Rahmen von thermischen Verletzungen (Verbrennung, Verbrühung) führte die hochdosierte intravenöse Anwendung von C1-Inaktivator-Konzentrat zu einer Durchbrechung der refraktären Hypotension sowie zu einer Rückbildung des generalisierten Ödems. Das verwandte therapeutische Anwendungsschema war wie folgt:

| | |
|---|---|
| initial | 5.000 E C1-Inaktivator i.v., |
| nach 12 Std. | 2.500 E C1-Inaktivator i.v., |
| nach weiteren 12 Std. | 1.500 E C1-Inaktivator i.v., |
| nach weiteren 12 Std. | 1.000 E C1-Inaktivator i.v. |

Bei Patienten mit Polytrauma führte die Anwendung von C1-Inaktivator-Konzentrat zur Korrektur der desolaten Kreislaufsituation. Die verwendeten Therapieschemata waren unterschiedlich, bewegten sich jedoch in der Größenordnung von 1.000 bis 6.000 E C1-Inaktivator-Konzentrat i. v. und wurden in der Regel repetitiv im 12-Std.-Abstand appliziert.

Bei Patienten mit therapeutisch oder prophylaktisch indizierter Knochenmarktransplantation wurde C1- Inaktivator-Konzentrat i. v. bei zwei Patienten eingesetzt. Das Dosierungsschema betrug:

| | |
|---|---|
| initial | 60 E/kg KG |
| nach 12 Std.: | 30 E/kg KG |
| nach 12 Std.: | 30 E/kg KG |
| nach 12 Std.: | 15 E/kg KG |
| nach 12 Std.: | 15 E/kg KG |
| nach 12 Std.: | 15 E/kg KG |
| nach 12 Std.: | 15 E/kg KG |

Bei diesen Patienten konnte das generalisierte Ödem, das beginnende "Veno-occlusive disease" sowie (in einem Fall) ein beginnendes Nierenversagen erfolgreich therapiert werden.

Bei Patienten, die sich einer Operation unter Bedingung des extrakorporalen Kreislaufs unterzogen haben, wurde C1-Inaktivator-Konzentrat bei insgesamt 56 Patienten i. v. appliziert. Bei 55 Patienten geschah dies im Rahmen einer klinischen Prüfung in der Indikation "Bypass Operation", bei einem Patienten, einem Neugeborenen, geschah dies im Rahmen einer therapeutischen "off label" Anwendung während einer sog. Transpositions-Operation der Aorta und A. pulmonalis. Die Resultate waren wie folgt:

Bei den erwachsenen Patienten, die C1-Inaktivator-Konzentrat im Rahmen einer Bypass-Operation erhielten, konnte bei denjenigen, die präoperativ an einer reduzierten Herzauswurfleistung litten, diese im peri- und postoperativen Verlauf deutlich gebessert werden.

Bei dem Neugeborenen trat im postoperativen Verlauf ein generalisiertes Ödem auf. Dieses konnte mit C1-Inaktivator-Konzentrat erfolgreich bekämpft werden und bildete sich zurück.

## Patentansprüche

1. Verwendung von C1-Inaktivator zur Herstellung eines Arzneimittels zur Prophylaxe und Therapie des Capillary Leak Syndroms (generalisierte Extravasation) und Kreislaufschocks (refraktäre Hypotension) bei schweren Verbrennungen oder Verbrühungen, bei Polytraumata, Operationen unter Bedingungen des extrakorporalen Kreislaufs oder bei Anwendung von Zytokinen, endogenen Mediatoren, gentechnologisch produzierten Mediator-Hybriden oder Wachstumsfaktoren im Rahmen der therapeutischen Anwendung dieser Substanzen sowie des Capillary Leak Syndroms und des Veno-occlusive Disease der Leber nach therapeutisch oder prophylaktisch indizierter Knochenmarktransplantation.

2. Verwendung nach Anspruch 1, dadurch gekennzeichnet, daß ein Mittel enthaltend 1 bis 5.000 E/kg KG/Tag, bevorzugt 5 bis 1.000 E/kg KG/Tag C1-Inaktivator hergestellt wird.

3. Verwendung nach Anspruch 1, dadurch gekennzeichnet, daß ein festes Mittel enthaltend C1-Inaktivator 1 bis 300.000 E/KG/Tag, bevorzugt 50 bis 60.000 E/KG/Tag hergestellt wird.

4. Verwendung nach Anspruch 1, dadurch gekennzeichnet, daß ein Mittel zur intravenösen, intramuskulären oder subkutanen Anwendung, besonders in Kombination mit galenischen Hilfsstoffen hergestellt wird.

5. Verwendung nach Anspruch 1, dadurch gekennzeichnet, daß ein Mittel zur peroralen oder rektalen Anwendung hergestellt wird, besonders in Kombination mit galenischen Hilfsstoffen hergestellt wird.
